# EUROPÄISCHE PATENTSCHRIFT

(11) **EP 1 685 896 B1**
(45) Veröffentlichungstag und Bekanntmachung des Hinweises auf die Patenterteilung: **30.07.2008**
(21) Anmeldenummer: 05027839.9
(22) Anmeldetag: 20.12.2005
(51) Int. Cl.: B01J 10/00, B01J 19/24, B01J 19/26, C08G 65/337, C07C 41/16, C07C 43/15

(54) **Gleichgewichtsreaktion und Gas/Flüssigreaktion im Schlaufenreaktor**
Equilibrium reactions and gas/liquid reaction in a loop reactor
Réactions d'équilibre et réaction gaz/liquide dans un réacteur en boucle

(30) Priorität: 08.01.2005 DE 102005001076
(43) Veröffentlichungstag der Anmeldung: 02.08.2006
(73) Patentinhaber: Evonik Goldschmidt GmbH, 45127 Essen (DE)
(72) Erfinder: Sellmann, Andreas, 40885 Ratingen (DE); Cramers, Peter, Dr., 4410 Liestal (CH); Kettenbach, Gerhard, Dr., 79639 Grenzach-Wyhlen (DE); Zellmer, Volker, Dr., 46240 Bottrop (DE)

(56) Entgegenhaltungen:
- EP-A- 0 419 419
- EP-A- 0 949 235
- WO-A-98/17381
- WO-A-20/04081082
- AT-B- 287 890
- DE-C1- 4 138 166

## Beschreibung

Die Erfindung betrifft ein Verfahren zur Veretherung schwersiedender Polyetheralkohole mit gleichzeitiger Durchführung zweier chemisch aufeinanderfolgender Reaktionen in einem Schlaufenreaktor, der einen Flüssigkeitsstrahl-Ejektor sowie ein geschlossenes Kondensationssystem aufweist.

Chemische Produktionsprozesse, die zwei oder mehrere chemisch aufeinanderfolgende, d.h. aufeinander aufbauende Reaktionen umfassen, bei denen eine Reaktion in einer Gleichgewichtsreaktion beispielsweise zwischen schwersiedenden flüssigen Edukten besteht und die darauf aufbauende Reaktion eine im Wesentlichen diffusionskontrollierte Reaktion ist, bei der das flüssige Zwischenprodukt der ersten Reaktion mit mindestens einem gasförmigen Reaktanden zum Endprodukt umgesetzt wird, werden großtechnisch derzeit in einer Weise durchgeführt, die beide Reaktionen räumlich, zeitlich und/oder prozesstechnisch voneinander trennt.

Beispielsweise werden bei einer großtechnischen Alkylierung von Polyetheralkoholen nach der Williamson-Methode in einem ersten Schritt (Schema 1) zunächst aus Polyetheralkohol und Natriummethanolat Natrium-Polyetheralkoholat und Methanol hergestellt.

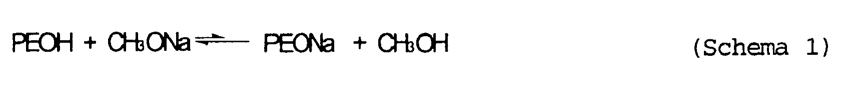

Das Reaktionsgleichgewicht dieser Gleichgewichtsreaktion liegt stark auf der Seite der Edukte und es muss daher durch effektives Abdestillieren des entstandenen Methanols auf die Produktseite verschoben werden. Dies gelingt jedoch nicht vollständig, da man aus verfahrenstechnischen Gründen im anzuwendenden Vakuum und wegen thermisch induzierter Umlagerungsreaktionen in der Höchsttemperatur auf Temperaturen unterhalb von 130 °C beschränkt ist. Vakuum von weniger als 20 mbar ist technisch nur mit unverhältnismäßig hohen Kosten zu realisieren. Des Weiteren erfordert die Gleichgewichtsreaktion einen hohen Überschuss an Natriummethanolat. Natriummethanolat kann grundsätzlich als methanolische Lösung oder auch in Pulverform eingesetzt werden. Bei der Verwendung methanolischer Lösung muss das Lösungsmittel Methanol ebenso wie das in der Reaktion entstehende Methanol möglichst effektiv entfernt werden, um das Reaktionsgleichgewicht auf die Produktseite zu verschieben. Leistungsfähige und kostspielige Destillationsverfahren wie der Einsatz von Dünnschichtverdampfern scheiden bei den hier anfallenden Mengen an Methanol aus. Natriummethanolat wird daher vielfach in Pulverform zugegeben, was allerdings den gefahrträchtigen Umgang mit stark hygroskopischem, selbstentzündlichem, ätzendem Pulver voraussetzt.

In einem zweiten Schritt wird im Stand der Technik das noch immer Edukte, insbesondere einen Überschuss an Natriummethanolat enthaltende Natriumpolyetheralkoholat in einem Gas-Flüssig-Reaktor mit gasförmigem Methylchlorid umgesetzt (Schema 2).

PEONa + CH₃Cl → PEOCH₃ + NaCl (Schema 2)

Überschüssiges Natriummethanolat reagiert dabei in einer unerwünschten Nebenreaktion zu Dimethylether, der als Abgas entsorgt werden muss. Methylchlorid (MeCl) reagiert äquimolar zum eingesetzten Natriummethanolat, d.h. ein hoher Überschuss an Natriummethanolat bedeutet einen großen Bedarf an Methylchlorid (Schema 3).

CH₃ONa + CH₃Cl → CH₃OCH₃ + NaCI (Schema 3)

Einen Sonderfall der dargestellten Verfahren des Standes der Technik stellt eine Alkoholatbildung gemäß Schema 1 dar, in der anstelle von Natriummethanolat Natronlauge NaOH eingesetzt wird.

Es ist bekannt, dass man die Ausbeute der beschriebenen und vergleichbarer Reaktionen durch hintereinandergeschaltete Methylierungsstufen in mehreren Reaktoren erhöhen kann, wobei sich an jede Methylierungsstufe ein Destillationsschritt anschließt, in dem Methanol bis zu einem Druck von 20 mbar bei 120 °C entfernt wird. Der apparative und verfahrenstechnische Aufwand ist jedoch erheblich.

Im Stand der Technik sind des Weiteren Schlaufenreaktoren bekannt, wobei wie beispielsweise in der EP-A-0 419 419 beschrieben, ein Gaskreislauf und ein Flüssigkeitskreislauf um den Reaktor angeordnet und miteinander über eine nach dem Venturi-Prinzip arbeitende Strahlsauger-Mischdüse (Flüssigkeitsstrahl-Ejektor) gekoppelt sind. Strahlsauger-Mischdüsen erzeugen große Phasenaustauschflächen zwischen Reaktionsgas und Flüssigkeit, wobei zwangsläufig das gasförmig zugeführte Reaktionsgas mit dem gegebenenfalls anwesenden Inertgas ständig intensiv gemischt und umgewälzt wird. Solche Reaktoren werden speziell für die Durchführung von Gas-Flüssig-Reaktionen eingesetzt, die sich durch eine sehr hohe Reaktionsgeschwindigkeit auszeichnen und daher nur durch den Stoffübergang von der Gasphase in die Flüssigphase bestimmt sind, deren Kinetik also diffusionskontrolliert ist, siehe beispielsweise WO-A-98/17381.

Des Weiteren ist bekannt, Gleichgewichtsreaktionen von schwersiedenden Stoffen, bei denen ein Leichtsieder entsteht, beispielsweise Veresterungsreaktionen im Bereich der Fettchemie in den oben beschriebenen Schlaufenreaktoren mit geschlossenem Gaskreislauf ablaufen zu lassen. Solche Reaktionen sind beispielsweise:

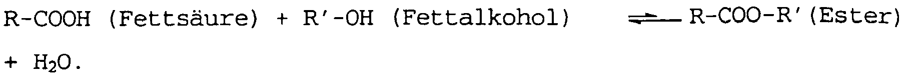

Die Edukte Fettsäure und Fettalkohol sowie der entstehende Ester sind schwersiedende Flüssigkeiten. Das Wasser entsteht bei den Prozessbedingungen dampfförmig, wird aus dem Gasraum des Reaktors mit Hilfe des Strahlsaugers über einen Kondensator gesaugt und kontinuierlich auskondensiert, um das Reaktionsgleichgewicht auf die gewünschte Seite zu verlagern.

Vor diesem Hintergrund bestand die Aufgabe, ein Verfahren zur Durchführung zweier chemisch aufeinanderfolgender Reaktionen in einem Reaktor zu entwickeln, nämlich
ein Verfahren zur Veretherung von schwersiedenden, flüssigen Alkyl- oder Alkenyl-gestarteten Polyetheralkoholen der allgemeinen Formel (1) 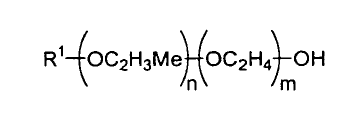 wobei
R¹ einen Alkyl- oder Alkenylrest mit bis zu 5 Kohlenstoffatomen darstellt,
Me für einen Methylrest steht,
n und m jeweils eine ganze Zahl im Bereich von 0 bis 90 darstellen und die Summe n + m im Bereich von 5 bis 90 liegt,
in einem Schlaufenreaktor, umfassend als Reaktion (I)
eine Deprotonierung des Polyetheralkohols mit einem schwersiedenden, flüssigen Alkalimethanolat, -ethanolat oder -propanolat, gegebenenfalls in Gegenwart eines leichtsiedenden Lösungsmittels, bei der im Wesentlichen ein schwersiedendes Alkalipolyetheralkoholat-Zwischenprodukt und mindestens ein leichtsiedender Alkohol als Nebenprodukt entsteht und als Reaktion (II)
eine Etherkupplung, bei der das flüssige Alkalipolyetheralkoholat-Zwischenprodukt mit gasförmigem Alkylhalogenid zum Endprodukt umgesetzt wird,
wobei, man die Reaktionen (I) und (II) - gegebenenfalls nach einer Startphase - gleichzeitig ablaufen lässt und der Schlaufenreaktor über einen über eine Strahlsauger-Mischdüse gekoppelten Gas-Flüssigkeits-Kreislauf verfügt, bei dem umgewälzte Flüssigphase in der Strahlsauger-Mischdüse mit neuzugeführtem (6) und/oder über den Strahlsauger aus dem Reaktionsraum über einen Kondensator (5) angesaugtem, gasförmigem Alkylhalogenid der Reaktion (II) vermischt wird und zur Reaktion durch einen Flüssigkeitsstrahl-Ejektor (2) dem Reaktionsraum zugeführt wird, wobei man im Kondensator das oder die leichtsiedende(n) Alkohol(e) der Reaktion (I) sowie gegebenenfalls leichtsiedendes Lösungsmittel über die Dauer des Verfahrens kontinuierlich kondensiert und ausschleust.

Durch die Kombination beider Reaktionen in dem erfindungsgemäßen Verfahren kann ein um ca. 5 bis 10 % höherer Gesamtumsatz erzielt werden, so dass bei der Herstellung von alkylierten Polyetheralkohlen der Überschuss an Natriummethanolat und somit auch an Methylchlorid und die Bildung von Dimethylether als Nebenprodukt aus der Reaktion des Natriummethanolatüberschusses mit Methylchlorid in der gleichen Größenordnung geringer gehalten werden können.

Im Einzelnen kann man das erfindungsgemäße Verfahren so ausgestalten, dass man eine Startphase vorsieht, in der man zunächst den schwersiedenden flüssigen Polyetheralkohol vorlegt, Alkalimethanolat, -ethanolat oder -propanolat, gegebenenfalls in leichtsiedendem Lösungsmittel, insbesondere Methanol, Ethanol oder Propanol gelöst hinzugibt und Reaktion (I) unter Kondensation und Ausschleusen des/der leichtflüchtigen Alkohol(e) und gegebenenfalls Lösungsmittel entweder im geschlossenen Kondensationsgaskreis oder durch Absaugen der Inertgase mittels einer Vakuumanlage über einen Kondensator bis zu einem Erreichen der verfahrens- und anlagetechnisch vorgegebenen Destillationsparameter, beispielsweise bis zu einem Umsatz von 90 bis 95 Mol-% bezogen auf den Polyetheralkohol durchführt. Zur Steigerung der Effizienz der Destillation kann am Ende der Destillation das oben beschriebene "Stickstoff-Strippen" durchgeführt werden.

Dabei ist es bevorzugt, eine Temperaturobergrenze von 120 °C nicht zu überschreiten.

Beispielsweise vermeidet man durch die Verwendung von 30 %iger methanolischer Natriummethanolat-Lösung die gefahrträchtige Handhabung von Natriummethanolat-Pulver. Außerdem kann das verwendete Lösungsmittel Methanol als Trägerbrennstoff in der Abgasverbrennungsanlage dienen.

Durch die Reduzierung des Natriumtnethanolatüberschusses um bis zu 60 % gegenüber dem Stand der Technik können im erfindungsgemäßen Verfahren der notwendige Rohstoffeinsatz und somit auch die Entstehung des Dimethylether reduziert werden.

Ideale Umsätze sind erzielt worden, wenn die Reaktion bis zu einer Restalkalizahl von kleiner 0,1 mg KOH/g durchführt wurde. Die Restalkalizahl ist dabei ein Maß die Umsetzung von Natriumionen des Polyether- oder Alkalialkoholats mit Methylchlorid, also nur für den Umsatz von Reaktion II. Sie wird durch Titration mit 0,1 n HCl gegen Phenolphthalein als Indikator bestimmt und in der Regel in mg KOH/g (Alkalizahl) umgerechnet. Bei vollständigem Umsatz aller Alkoholatgruppen betrüge die Alkalizahl 0 mg KOH/g. Entscheidend für den Gesamtumsatz über beide Reaktionen ist der Rest an Polyetheralkohol. Da die Bestimmung der so genannten Rest-OH-Zahl analytisch aufwendig ist, wird sich im Folgenden stets auf die Restalkalizahl bezogen.

Ein entsprechend geeigneter Schlaufenreaktor, der über einen zuschaltbaren über eine Strahlsauger-Mischdüse gekoppelten GasFlüssigkeits-Kreislauf verfügt, ist an sich im Stand der Technik bekannt und wird beispielsweise für die Quaternisierung von Aminen mit Methylchlorid eingesetzt. Die gleichzeitige Reaktionsführung einer schnellen Gas-Flüssigreaktion mit einer vorgelagerten Gleichgewichtsreaktion unter kontinuierlicher Abdestillation des leichtsiedenden Nebenproduktes ist jedoch bislang im Stand der Technik nicht bekannt.

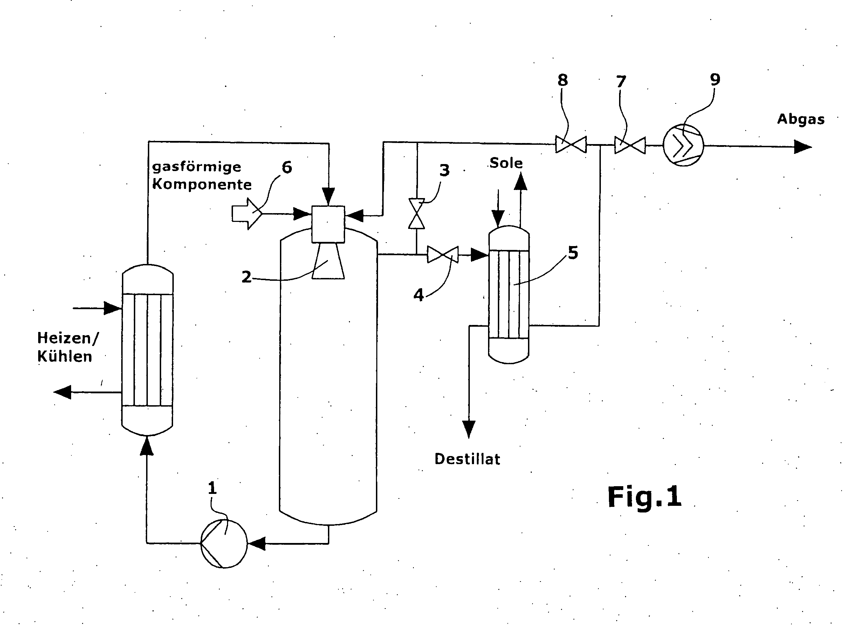

Fig. 1 zeigt einen solchen Schlaufenreaktor. Der Reaktor verfügt über einen Reaktionsraum und einen Umwälzkreislauf, der mit einer Umwälzpumpe 1 über einen Wärmeaustauscher betrieben wird. Die umgewälzte Flüssigkeit wird in dem gezeigten Schlaufenreaktor in einer Strahlsauger-Mischdüse 2 mit angesaugtem, gasförmigem Edukt 6 vermischt und in den Reaktionsraum zurückgefördert. Zudem besteht eine mit einem Ventil 3 verschließbare Verbindung des Gasraumes des Reaktionsraumes mit der Ansaugöffnung der Strahlsauger-Mischdüse 2, über die die Gasphase angesaugt, mit gasförmigem Edukt 6 und umgewälzter Flüssigphase vermischt und in den Reaktionsraum zurückgefördert werden kann. In diese Verbindung kann über ein Ventil 4 ein geschlossener Gaskreislauf mit einem Kühler/Wärmetauscher 5 und je einer Entnahmestelle für Destillat und Abgas zugeschaltet werden. Der Wärmetauscher 5 kann hierbei durch Abkühlen des angesaugten Gases unterhalb des Siedepunktes als Kondensator 5 betrieben werden. Durch Regelung der Kondensationstemperatur wird dabei festgelegt, in welcher Zusammensetzung die Abgasbestandteile auskondensieren.

Solche Schlaufenreaktoren haben für die Umsetzung von Gasen mit Flüssigkeiten hinsichtlich Betriebssicherheit und Produktqualität Vorteile, da lokale Inhomogenitäten bezüglich Konzentrationen und Temperatur effizient vermieden werden können.

Die Strahlsauger-Mischdüse (2) ist eine nach dem Venturi-Prinzip arbeitende Mischdüse.

### Ausführungsbeispiele:

### Beispiel 1:

In einer Vorrichtung gemäß Fig. 1 mit einem Schlaufenreaktor mit 50 1 Fassungsvermögen im Reaktionsraum wurden zunächst 40 kg eines schwersiedenden Polyetheralkohols vorgelegt. Bei dem eingesetzten Polyetheralkohol handelte es sich um einen alkenylgestarteten Polyether in der Form

R¹-(OC₂H₃M_{O})ₙ-(OC₂H₄)ₘ-OH,

wobei
- R¹: ein Alkenylrest mit 3 C-Atomen ist und
- n: 13 und
- m: 4
betragen.

Zu dem Polyetheralkohol wurden daraufhin 15 kg 30 %iger Natriummethanolat-Lösung in Methanol in den Reaktor zudosiert. Im geschlossenen Kondensationskreislauf mit geschlossenem Ventil 3 und geöffnetem Ventil 4 wurden die Reaktionspartner bei 110 °C entsprechend der Gleichgewichtsreaktion I umgesetzt, wobei im Kondensator 5 Methanol soweit auskondensiert wurde, wie es die Kondensationstemperatur von -15 °C erlaubte (Startphase). Entsprechend der Kondensationstemperatur stellte sich ein Methanolpartialdruck in der Gasphase bzw. über das Phasengleichgewicht ein Methanol-Restgehalt in der Flüssigphase ein, der mit einem Umsatz der Gleichgewichtsreaktion I in Höhe von 90 bis 95 Mol-% korrespondierte. Nachdem über diese Fahrweise kein Destillat im Kondensator 5 mehr anfiel, wurden die Ventile 8 geschlossen und 7 geöffnet und mittels Vakuumanlage 9 ein Endvakuum von kleiner als 20 mbar erreicht. Um die Destillationsbedingungen für Methanol noch weiter zu verbessern, wurde Stickstoff während der Vakuumdestillation über den Strahlsauger 2 etwa 15 min lang zugegeben und so der Methanolpartialdruck in der Gasphase weiter reduziert.

Danach wurde das Alkoholat mit Methylchlorid über die Strahlsauger-Mischdüse 2 mit der umgewälzten Flüssigphase innig vermischt und in den Reaktionsraum zurückgefördert. Die Temperatur im Reaktionsraum betrug 110 bis 130 °C. Dabei erfolgte die Williamsonsche Ethersynthese zum gewünschten Endprodukt PEOCH₃ und Kochsalz gemäß Reaktionsschema 2. Der Endpunkt der Reaktion wurde bestimmt durch das Erreichen eines Plateaus der Alkalizahl.

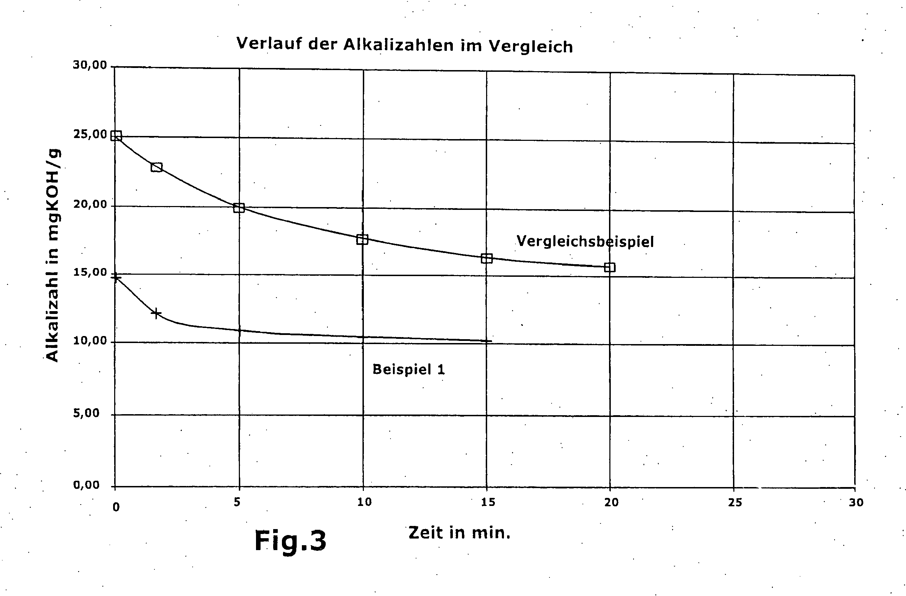

Das Kochsalz verblieb zunächst in feindispergierter Form im Produkt, das in einen Rührbehälter überführt wurde, zur Neutralisation mit Phosphorsäure homogenisiert wurde und über eine Rahmenfilterpresse filtriert wurde. Mit dem Verfahren wurde nach 15 min eine Alkalizahl von etwa 10 mg KOH/g erzielt (Fig. 3).

Vergleichsbeispiel:
Das Verfahren wurde analog Beispiel 1 mit dem Unterschied durchgeführt, dass während der Methylierungsreaktion, also nach der Startphase, der Kondensator im geschlossenen Gaskreislauf durch Schließen des Ventils 4 und Öffnen des Ventils 3 umgangen wurde. Der Endpunkt der Reaktion wurde bestimmt durch das Erreichen eines Plateaus der Alkalizahl. Es wurde nach 20 min eine Alkalizahl von etwa 16 mg KOH/g erzielt (Fig. 3).
Durch die in Beispiel 1 angewandte erfindungsgemäße Verfahrensweise konnten Gesamtumsätze von Polyetheralkohol (PEOH) zum methylierten Polyether (PE-OCH₃) von 90 bis nahezu 100 % erzielt werden. Nach dem Stand der Technik übliche Methylierungsgrade liegen bei etwa 80 bis 90 %.

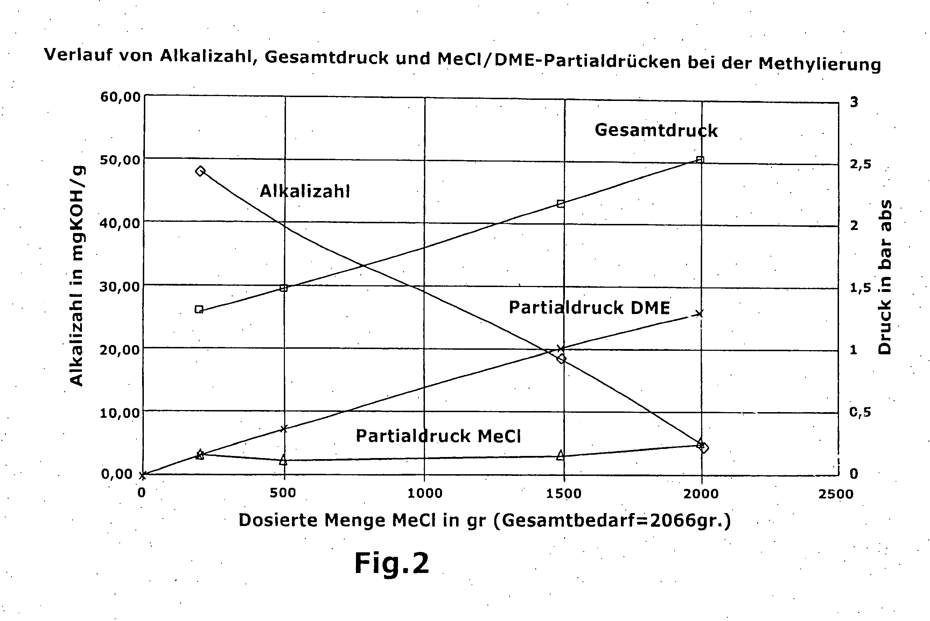

Die Fig. 2 und 3 zeigen die Effektivität der Umsetzung von flüssigem Polyetheralkoholat mit gasförmigem Methylchlorid im Schlaufenreaktor mit Strahlsauger. Methylchlorid reagiert in diesem System so schnell ab, dass der Hold-up bzw. Partialdruck an Methylchlorid in der Gasphase extrem gering ist (Fig. 2) und bei Unterbrechung der Methylchlorid-Dosage der Methylchlorid-Hold-up im Vergleich zu einem Rührreaktor sehr schnell (in weniger als fünf Minuten) abreagiert (Fig. 3).

## Patentansprüche

1. Verfahren zur Veretherung von schwersiedenden, flüssigen Alkyl- oder Alkenyl-gestarteten Polyetheralkoholen der allgemeinen Formel (1) 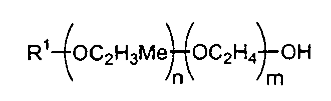 wobei
R¹ einen Alkyl- oder Alkenylrest mit bis zu 5 Kohlenstoffatomen darstellt,
Me für einen Methylrest steht,
n und m jeweils eine ganze Zahl im Bereich von 0 bis 90 darstellen und die Summe n + m im Bereich von 5 bis 90 liegt,
in einem Schlaufenreaktor, umfassend als Reaktion (I) eine Deprotonierung des Polyetheralkohols mit einem schwersiedenden, flüssigen Alkalimethanolat, -ethanolat oder -propanolat, gegebenenfalls in Gegenwart eines leichtsiedenden Lösungsmittels, bei der im Wesentlichen ein schwersiedendes Alkalipolyetheralkoholat-Zwischenprodukt und mindestens ein leichtsiedender Alkohol als Nebenprodukt entsteht und
als Reaktion (II) eine Etherkupplung, bei der das flüssige Alkalipolyetheralkoholat-Zwischenprodukt mit gasförmigem Alkylhalogenid zum Endprodukt umgesetzt wird,
**dadurch gekennzeichnet, dass** man die Reaktionen (I) und (II) - gegebenenfalls nach einer Startphase - gleichzeitig ablaufen lässt und der Schlaufenreaktor über einen über eine Strahlsauger-Mischdüse gekoppelten Gas-Flüssigkeits-Kreislauf verfügt, bei dem umgewälzte Flüssigphase in der Strahlsauger-Mischdüse mit neuzugeführtem (6) und/oder über den Strahlsauger aus dem Reaktionsraum über einen Kondensator (5) angesaugtem, gasförmigem Alkylhalogenid der Reaktion (II) vermischt wird und zur Reaktion durch einen Flüssigkeitsstrahl-Ejektor (2) dem Reaktionsraum zugeführt wird, wobei man im Kondensator das oder die leichtsiedende(n) Alkohol(e) der Reaktion (I) sowie gegebenenfalls leichtsiedendes Lösungsmittel über die Dauer des Verfahrens kontinuierlich kondensiert und ausschleust.

2. Verfahren gemäß Anspruch 1, **dadurch gekennzeichnet, dass** man eine Startphase vorsieht, in der man zunächst den schwersiedenden flüssigen Polyetheralkohol vorlegt, Alkalimethanolat, -ethanolat oder -propanolat, gegebenenfalls in leichtsiedendem Lösungsmittel, insbesondere Methanol, Ethanol oder Propanol gelöst hinzugibt und Reaktion (I) unter Kondensation und Ausschleusen des/der leichtflüchtigen Alkohol(e) und gegebenenfalls Lösungsmittel entweder im geschlossenen Kondensationsgaskreis oder durch Absaugen der Inertgase mittels einer Vakuumanlage über einen Kondensator bis zum Erreichen der verfahrens- und anlagetechnisch vorgegebenen Destillationsparameter durchführt.

3. Verfahren gemäß Anspruch 1 oder 2, **dadurch gekennzeichnet, dass** man eine Temperaturobergrenze von 120 °C nicht überschreitet.

4. Verfahren gemäß einem der Ansprüche 1 bis 3, **dadurch gekennzeichnet, dass** man die Reaktion bis zu einer Restalkalizahl von kleiner als 0,1 mg KOH/g durchführt.

## Claims

1. Process for etherifying high-boiling, liquid alkyl- or alkenyl-started polyether alcohols of the general formula (1) 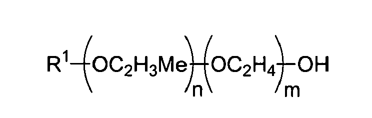 where
R¹ is an alkyl- or alkenyl radical having up to 5 carbon atoms,
Me is a methyl radical,
n and m are each an integer in the range from 0 to 90 and the sum of n + m is in the range from 5 to 90,
in a loop reactor, comprising, as reaction (I)
a deprotonation of the polyether alcohol, with a high-boiling, liquid alkali metal methylate, ethoxide or propoxide, optionally in the presence of a low-boiling solvent to form substantially a high-boiling alkali metal polyether alkoxide intermediate and at least one low-boiling alcohol as a by-product and,
as reaction (II), an ether coupling in which the liquid alkali metal polyether alkoxide intermediate is reacted with gaseous alkyl halide to give the end product,
**characterized in that** reactions (I) and (II), optionally after a start phase, are allowed to proceed simultaneously and the loop reactor has a gas-liquid circulation system coupled via an ejector mixing nozzle, in which circulated liquid phase is mixed in the ejector mixing nozzle with newly supplied gaseous alkyl halide of reaction (II) (6) and/or gaseous alkyl halide of reaction (II) aspirated by means of the ejector from the reactor via a condenser (5), and fed to the reactor by a liquid jet ejector (2) for the reaction, the low-boiling alcohol(s) of reaction (I) and also any low-boiling solvent being condensed and discharged in the condenser continuously over the duration of the process.

2. Process according to Claim 1, **characterized in that** a start phase is provided in which the high-boiling liquid polyether alcohol is initially charged, alkali metal methylate, ethoxide or propoxide, optionally dissolved in low-boiling solvent, especially methanol, ethanol or propanol, is added, and reaction (I) is carried out with condensation and discharge of the volatile alcohol(s) and any solvent, either in a closed condensation gas circuit or by aspirating the inert gases by means of a vacuum unit via a condenser, until the distillation parameters predefined according to the process and plant technology have been attained.

3. Process according to Claim 1 or 2, **characterized in that** an upper temperature limit of 120°C is not exceeded.

4. Process according to one of Claims 1 to 3,
**characterized in that** the reaction is carried out down to a residual alkali number of less than 0.1 mg KOH/g.

## Revendications

1. Procédé en vue de l'éthérification de polyétheralcools fluides, à point d'ébullition élevé, à amorce alkyle ou alcényle, de la formule générale (1) 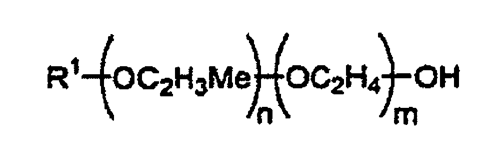 dans laquelle
R¹ représente un radical alkyle ou alcényle ayant jusqu'à 5 atomes de carbone,
Me représente un radical méthyle,
n et m représentent à chaque fois un nombre entier dans le domaine de 0 à 90 et la somme n + m se situe dans le domaine de 5 à 90,
dans un réacteur à boucle, comprenant en tant que réaction (I), une déprotonation du polyétheralcool à l'aide d'un méthanolate, d'un éthanolate ou d'un propanolate alcalin fluide, à point d'ébullition élevé, le cas échéant, en présence d'un solvant à point d'ébullition faible, lors de laquelle se forment, pour l'essentiel, un produit intermédiaire de polyétheralcoolate alcalin à point d'ébullition élevé et au moins un alcool à point d'ébullition faible en tant que produit secondaire et
en tant que réaction (II), un couplage éther, lors duquel le produit intermédiaire de polyétheralcoolate alcalin fluide entre en réaction avec un halogénure d'alkyle gazeux pour former le produit final,
**caractérisé en ce que** l'on laisse les réactions (I) et (II) - le cas échéant, après une phase d'amorçage - se dérouler simultanément et **en ce que** le réacteur à boucle dispose d'un circuit fermé gaz-fluide couplé par l'intermédiaire d'un dispositif aspirant à jet - d'une buse de mélange, auquel cas la phase fluide mise en circulation est mélangée dans le dispositif aspirant à jet - la buse de mélange à de l'halogénure d'alkyle gazeux de la réaction (II), nouvellement acheminé (6) et/ou aspiré par le dispositif aspirant à jet hors de l'espace de réaction à travers un condenseur (5) et acheminée à l'espace de réaction en vue de la réaction à l'aide d'un éjecteur à jet liquide (2), le ou les alcools à point d'ébullition faible de la réaction (I) ainsi que, le cas échéant, le solvant à point d'ébullition faible étant condensés dans le condenseur et évacués en continu sur l'ensemble de la durée du procédé.

2. Procédé selon la revendication 1, **caractérisé en ce que** l'on prévoit une phase d'amorçage, dans laquelle l'on introduit initialement tout d'abord le polyéther alcool fluide à point d'ébullition élevé, **en ce que** l'on ajoute le méthanolate, l'éthanolate ou le propanolate alcalin, sous forme dissoute le cas échéant, dans un solvant à point d'ébullition faible, en particulier le méthanol, l'éthanol ou le propanol, et **en ce que** l'on effectue la réaction (I) sous condensation et évacuation du ou des alcools à point d'ébullition faible et, le cas échéant, du solvant, soit dans le circuit gazeux de condensation fermé, soit par aspiration des gaz inertes à l'aide d'une installation sous vide par l'intermédiaire d'un condenseur jusqu'à obtention des paramètres de distillation prescrits du point de vue du procédé et de l'installation.

3. Procédé selon la revendication 1 ou 2, **caractérisé en ce que** l'on ne dépasse pas une limite de température de 120°C.

4. Procédé selon l'une quelconque des revendications 1 à 3, **caractérisé en ce que** l'on effectue la réaction jusqu'à obtenir un indice alcalin résiduel inférieur à 0,1 mg de KOH/g.
